# EUROPÄISCHE PATENTSCHRIFT

(11) **EP 3 443 942 B1**
(45) Veröffentlichungstag und Bekanntmachung des Hinweises auf die Patenterteilung: **07.12.2022**
(21) Anmeldenummer: 17186732.8
(22) Anmeldetag: 17.08.2017
(51) Int. Cl.: A61F 13/58, C09J 7/38

(54) **KLEBENDES HYGIENEARTIKELVERSCHLUSSBAND, HALBZEUGBANDMATERIAL ZUR HERSTELLUNG EINES KLEBENDEN HYGIENEARTIKELVER-SCHLUSS-BANDS UND MIT EINEM HYGIENEARTIKELVERSCHLUSSBAND VERSCHLOSSENER ODER VERSCHLIESSBARER HYGIENE-ARTIKEL**
ADHESIVE HYGIENIC ITEM CLOSURE TAPE, SEMIFINISHED TAPE MATERIAL OF MANUFACTURING AN ADHESIVE HYGIENIC ITEM CLOSURE TAPE AND HYGIENIC ITEM WHICH CAN BE SEALED OR CLOSED WITH HYGIENIC ITEM CLOSURE TAPE
BANDE DE FERMETURE ADHÉSIF POUR ARTICLE D'HYGIÈNE, MATÉRIAU DE BANDE SEMI-FINI DESTINÉ À LA FABRICATION D'UNE BANDE DE FERMETURE ADHÉSIF POUR ARTICLE D'HYGIÈNE ET ARTICLE D'HYGIÈNE POUVANT ÊTRE FERMÉ AU MOYEN D'UNE BANDE DE FERMETURE ADHÉSIF POUR ARTICLE D'HYGIÈNE

(43) Veröffentlichungstag der Anmeldung: 20.02.2019
(73) Patentinhaber: Lohmann-koester GmbH & Co. KG, 96146 Altendorf (DE)
(72) Erfinder: Schmidt, Dominik, 96146 Altendorf (DE); Neugebauer, Robert, 96146 Altendorf (DE)
(74) Vertreter: Reuther, Martin

(56) Entgegenhaltungen:
- EP-A1- 0 520 087
- EP-A1- 0 755 665
- EP-A1- 2 072 595
- EP-A2- 2 123 727
- WO-A1-2012/129428

## Beschreibung

Die Erfindung betrifft ein klebendes Hygieneartikelverschlussband, ein Halbzeugbandmaterial zur Herstellung eines klebenden Hygieneartikelverschlussbands und einen mit einem Hygieneartikelverschlussband verschlossener oder verschließbarer Hygieneartikel mit den Merkmalen der Oberbegriffe der unabhängigen Ansprüche.

Derartige Hygieneartikelverschlussbänder sind in vielfältiger Hinsicht aus dem Stand der Technik bekannt. So finden sich derartige Hygieneartikelverschlussbänder beispielsweise zum wiederverschließbaren Verschließen von Umverpackungen für Papiertaschentüchern oder Damenbinden bzw. bei Windelanwendungen. Auch sind derartige Hygieneartikelverschlussbänder beispielsweise aus der EP 1 084 204 B1, der EP 2 072 595 A1, der EP 2 431 435 A2 oder der WO 2012/129428 A1 bekannt. Ebenso die EP 1 092 759 A2 offenbart derartige Hygieneartikelverschlussbänder.

Entsprechende klebende Hygieneartikelverschlussbänder weisen in der Regel ein Trägerband auf, welches eine klebstofftragende Vorderseite und eine Rückseite aufweist, wobei an der Vorderseite wenigstens eine Klebstoffschicht, die einen ersten Klebefunktionsbereich und einen zweiten Klebefunktionsbereich bildet, vorgesehen ist. Sie werden häufig aus einem Bandmaterial als Halbzeug hergestellt, indem entsprechende klebende Hygieneartikelverschlussbänder von diesem Halbzeugbandmaterial senkrecht zu dessen Banderstreckungsrichtung abgetrennt werden, sodass sich in einer Längserstreckungsrichtung längs erstreckende klebende Hygieneartikelverschlussbänder bereitstellen lassen. Hierbei weisen die entsprechenden Halbzeugbandmaterialien ebenfalls ein Trägerband auf, welches eine Klebstoff tragende Vorderseite und eine Rückseite aufweist, wobei an der Vorderseite wenigstens eine Klebstoffbahn, die einen ersten Klebefunktionsbereich und eine zweiten Klebefunktionsbereich bildet, vorgesehen ist. Entsprechende Hygieneartikel, welche mit diesen Hygieneartikelverschlussbändern verschlossen bzw. verschließbar ausgestaltet werden sollen, weisen zumeist eine erste Verschlusszone und eine zweite Verschlusszone auf, die miteinander zu verschließen sind. Das entsprechende Hygieneartikelverschlussband wird in der Regel an einer der Verschlusszonen permanent befestigt, um dann bis zu der zweiten Verschlusszone zu reichen und den Verschluss schließen zu können. Je nach konkreter Ausgestaltung kann dieser Verschluss derart fest ausgebildet sein, dass der Verschluss lediglich durch Zerstören getrennt werden kann. Häufig wird jedoch die Verbindung zu der zweiten Verschlusszone lösbar sein. Da in der Regel der mit der zweiten Verschlusszone verbundene Bereich des Hygieneartikelverschlussbands durch den Benutzer zumindest geöffnet oder auch geschlossen werden kann, wird dieser Bereich auch Benutzerbereich genannt, während der permanent mit der ersten Verschlusszone des Hygieneartikels verbundene Bereich des Hygieneartikelverschlussbands auch Permanentbereich genannt wird. Hierbei versteht es sich, dass der Permanentbereich des Hygieneartikelverschlussbands ggf. auch unter etwas größerem Aufwand bzw. unter Zerstörung der beteiligten Baugruppen geöffnet werden könnte.

Insofern zeichnen sich klebende Hygieneartikelverschlussbänder dadurch aus, dass sie zumindest zwei Klebefunktionsbereiche, die entsprechende funktionale Bereiche des Hygieneartikelverschlussbands darstellen, umfassen, die dazu geeignet und dafür bestimmt sind, einen Verschluss bei einem Hygieneartikel zu bilden und durch diese Verklebungen den Verschluss bereit zu stellen.

Es ist Aufgabe vorliegender Erfindung, klebende Hygieneartikelverschlussbänder, Halbzeugbandmaterial zur Herstellung klebender Hygieneartikelverschlussbänder und mit einem Hygieneartikelverschlussband verschlossene oder verschließbare Hygieneartikel bereitzustellen, bei denen die Hygieneartikelverschlussbänder auch bei minimalen Abmessungen betriebssicher an einem Hygieneartikel maschinell zu befestigen ist.

Die Aufgabe der Erfindung wird durch klebende Hygieneartikelverschlussbänder, Halbzeugbandmaterial zur Herstellung klebender Hygieneartikelverschlussbänder und mit einem Hygieneartikelverschlussband verschlossene oder verschließbare Hygieneartikel mit den Merkmalen der unabhängigen Ansprüche gelöst. Weitere, ggf. auch unabhängig hiervon, vorteilhafte Ausgestaltungen finden sich in den Unteransprüchen sowie der nachfolgenden Beschreibung.

Hierbei geht die Erfindung von der Grunderkenntnis aus, dass bei der maschinellen Befestigung der Hygieneartikelverschlussbänder trotz der geringeren peel-off-Kräfte eines der Klebefunktionsbereiche eine betriebssichere Befestigung an dem Hygieneartikel bzw. an dessen Umverpackungen oder an sonstigen Baugruppen möglich ist, da durch dessen größerer Ausgestaltung dennoch ein betriebssicherer Sitz bzw. ein Aufspringen während der maschinellen Bearbeitung verhindert werden kann. Andererseits kann der Klebefunktionsbereich mit den höheren peel-off-Kräften entsprechend klein ausgebildet werden, was hinsichtlich möglichst minimaler Abmessung von Vorteil ist. Bei geeigneter Wahl bzw. Ausgestaltung der Klebefunktionsbereiche ist es dann möglich, beispielsweise auch an sehr dünnen Folien, welche durchaus 20 µm unterschreiten können, einerseits betriebssicher permanent und andererseits aber auch in dem Klebefunktionsbereich mit den geringeren peel-off-Kräften lösbar mit der Folie zu verbinden, ohne dass beim Öffnen des Hygieneartikelverschlussbands eine Zerstörung der zweiten Verschlusszone befürchtet werden muss. Dieses gilt dementsprechend auch für andere Oberflächen, wie beispielsweise besonders empfindliche Non-Woven bei denen verhindert werden soll, das beim Öffnen zu viele der Oberflächenbestandteile bzw.- Filamente durch den entsprechenden Klebefunktionsbereich ausgerissen werden. Gleichwohl kann das entsprechende Hygieneartikelverschlussband maschinell an den entsprechenden Oberflächen ohne ein Aufspringen befestigt werden, obgleich unter diesen Oberflächen evtl. sehr weiche Körper, wie beispielsweise Windelbestandteile oder eine Damenbinde bzw. ein Stapel Papiertaschentücher zu finden ist.

Nach Durchlaufen der entsprechenden Maschinen werden die entsprechenden Hygieneartikel in der Regel in größeren Gebinden gelagert, wobei die dann auftretenden, verhältnismäßig gleichmäßigen Kräfte weniger kritisch hinsichtlich eines unbeabsichtigten Aufspringens der Hygieneartikelverschlussbänder sind. Hierbei können insbesondere die Klebstoffe in aller Ruhe ausheilen und eine Verbindung mit den jeweiligen Verschlusszonen eingehen, sodass hiernach ein Aufspringen noch unwahrscheinlicher ist. Durch die Unterschiede in den peel-off-Kräften und den Abmessungen der Klebefunktionsbereiche kann bei minimalen Abmessungen insbesondere zu den kritischen Zeiten während oder unmittelbar nach einer Applikation der Hygieneartikelverschlussbänder an dem entsprechenden Hygieneartikel eine betriebssichere Befestigung derselben gewährleistet werden.

So kann sich ein klebendes Hygieneartikelverschlussband mit einem Trägerband, welches eine klebstofftragende Vorderseite und eine Rückseite aufweist, wobei an der Vorderseite wenigstens eine Klebstoffschicht, die einen ersten Klebefunktionsbereich und einen zweiten Klebefunktionsbereich bildet, vorgesehen ist, dadurch auszeichnen, dass peel-off-Kräfte des ersten Klebefunktionsbereichs der beiden Klebefunktionsbereiche größer als peel-off-Kräfte des zweiten Klebefunktionsbereichs der beiden Klebefunktionsbereiche sind und dass der erste Klebefunktionsbereich kleiner als der zweite Klebefunktionsbereich ausgebildet ist, um auf diese Weise eine betriebssichere maschinelle Befestigung des Hygieneartikelverschlussbands auch bei minimalen Abmessungen zu gewährleisten.

Ebenso kann ein Halbzeugbandmaterial zur Herstellung eines sich entlang einer senkrecht zu einer Banderstreckungsrichtung des Halbzeugbandmaterials ausgerichteten Längserstreckungsrichtung längs erstreckenden klebenden Hygieneartikelverschlussbands eine betriebssichere maschinelle Befestigung des hieraus bereitgestellten Hygieneartikelverschlussbands auch bei minimalen Abmessungen an einem Hygieneartikel gewährleisten, wenn das Halbzeugbandmaterial ein Trägerband umfasst, welches eine Klebstoff tragende Vorderseite und eine Rückseite aufweist, wobei an der Vorderseite wenigstens eine Klebstoffbahn, die einen ersten Klebefunktionsbereich und einen zweiten Klebefunktionsbereich bildet, vorgesehen ist, und wenn sich das Halbzeugbandmaterial dadurch auszeichnet, dass peel-off-Kräfte des ersten Klebefunktionsbereichs der beiden Klebefunktionsbereiche größer als peel-off-Kräfte des zweiten Klebefunktionsbereichs der beiden Klebefunktionsbereiche sind und dass der erste Klebefunktionsbereich in Längserstreckungsrichtung schmaler als der zweite Klebefunktionsbereich ausgebildet ist.

Durch entsprechende klebende Hygieneartikelverschlussbänder können dann mit einem Hygieneartikelverschlussband verschlossene oder verschließbare Hygieneartikel, die eine erste Verschlusszone und eine zweite Verschlusszone, die miteinander zu verschließen sind, sowie das Hygieneartikelverschlussband umfassen, welches mit seinem einen Permanentbereich bildenden ersten Klebefunktionsbereich permanent mit der ersten Verschlusszone und mit seinem einen Benutzerbereich bildenden zweiten Klebefunktionsbereich mit der zweiten Verschlusszone verbunden, verbindbar oder lösbar verbunden sind, bereitgestellt werden, die sich dadurch auszeichnen, dass die beiden Klebefunktionsbereiche des Hygieneartikelverschlussbands gemeinsam mit einer der beiden Verschlusszonen verbindbar bzw. verbunden sind. Durch eine derartige Ausgestaltung des entsprechenden Hygieneartikels kann insbesondere maschinellen Ungenauigkeiten Rechnung getragen werden und betriebssicher sichergestellt sein, dass der den Permanentbereich bildende funktionale Bereich nicht mit der zweiten Verschlusszonen in Kontakt kommen kann.

Hygieneartikelverschlussbänder können unterschiedliche funktionale Bereiche aufweisen, die - wie schon die Begrifflichkeit festlegt - unterschiedliche Funktionen erfüllen. So können beispielsweise funktionale Bereiche zur Bildung eines Fingerlifts oder Bereiche, die einer permanenten oder lösbaren Verbindung dienen, aber auch Zwischenbereiche, mit denen Abstände geschaffen werden, um beispielsweise Produktionssicherheit oder ein einfaches Ergreifen bzw. ein zielgerichtetes Positionieren von Fasteningbereichen zu ermöglichen, vorgesehen sein. Beispiele für derartige Ausgestaltungen finden sich unter anderen in der EP 1 092 759 A2, wobei hier etwaige Unterschiede in den peel-off-Kräften allenfalls erahnt werden können, da hier explizit Klebstoffe mit unterschiedlich Elastizitätsmodulen zur Anwendung kommen sollen. Insbesondere können derartige funktionale Bereiche durch entsprechend ausgestaltete Klebefunktionsbereiche dargestellt werden, mittels derer sich gezielt beispielsweise Permanentbereiche oder auch Fasteningbereiche bzw. Benutzerbereiche bereitstellen lassen. Dieses gilt insbesondere in Abgrenzung zu den Gegenständen der EP 1 084 204 B1, der EP 2 072 595 A1, der EP 2 431 435 A2 oder der WO 2012/129428 A1, nach welchen ein einziger Klebefunktionsbereich durch ein Nebeneinander mehrerer Klebstoffstreifen, die jeweils in sich konstante Klebstoffeigenschaften jedoch einander abwechselnd jeweils unterschiedliche Klebstoffeigenschaften aufweisen, dargestellt wird, um auf diese Weise durch die sequentielle und abwechselnde Anordnung die Klebstoffeigenschaften des jeweiligen gesamten Klebefunktionsbereichs insgesamt entsprechen anpassen zu können. Entsprechendes offenbart auch die EP 2 123 727 A2 für Klebebänder im Baubereich, bei denen über die gesamte Länge des Bandes durch unterschiedliche Klebstoffstreifen das Gesamtklebeverhalten des Bandes modifiziert werden soll, wobei hier, im Gegensatz zu Hygieneartikelverschlussbänder mit verhältnismäßig großen Klebstoffstreifen gearbeitet wird. Wie unmittelbar nachvollziehbar, gelingt eine Anpassung der Klebstoffeigenschaften des jeweiligen Klebefunktionsbereichs bzw. Klebebandes durch die sequentielle und abwechselnde Anordnung der Klebstoffstreifen nur, wenn die Klebstoffstreifen ausreichend schmal und eng nebeneinander ausgebildet sind, da ansonsten keine konstante Verhaltensweise des hierdurch gebildeten Klebefunktionsbereichs bzw. Klebebands gewährleistet werden kann.

Es versteht sich, dass ein klebendes Hygieneartikelverschlussband bzw. ein entsprechendes Halbzeugbandmaterial dann zumindest zwei funktionale Bereiche aufweisen kann, die insbesondere durch den ersten Klebefunktionsbereich und durch den zweiten Klebefunktionsbereich bereitgestellt werden.

In der Regel wird das Halbzeugbandmaterial in linear laufenden Maschinen gefertigt, sodass die Banderstreckungsrichtung der Maschinenrichtung, also der Richtung, in welcher das Band die entsprechende Fertigungsmaschine durchläuft, entspricht. Von dem Halbzeugbandmaterial kann dann senkrecht zur Banderstreckungsrichtung jeweils streifenartig das klebende Hygieneartikelverschlussband abgetrennt werden, welches dann eine Längserstreckungsrichtung aufweist, die senkrecht zu der Banderstreckungsrichtung bzw. zu der Maschinenrichtung ausgerichtet ist.

Insbesondere kann das Halbzeugbandmaterial einnutzig bzw. mehrnutzig ausgebildet sein. Bei einer Einnutzigkeit wird durch jeden parallel zur Längserstreckungsrichtung bzw. senkrecht zur Banderstreckungsrichtung durchgeführten Trennvorgang genau ein entsprechendes Hygieneartikelverschlussband bereitgestellt. Bei einer mehrnutzigen Ausgestaltung erzeugt eine entsprechende Trennung mehrere Hygieneartikelverschlussbänder, die dann noch senkrecht zur Längserstreckungsrichtung bzw. in Banderstreckungsrichtung voneinander getrennt werden müssen, wobei es sich versteht, dass letzterer Trennvorgang auch vor bzw. während dem Trennen parallel zur Längserstreckungsrichtung bzw. senkrecht zur Banderstreckungsrichtung erfolgen kann. Je nach konkreter Umsetzung kann bei mehrnutzigen Halbzeugbandmaterialien ein Trennen auch versetzt erfolgen, wenn beispielsweise die Trennlinie, mit welcher das Halbzeugbandmaterial in Maschinenrichtung getrennt ist, in Abhängigkeit von dem Banddurchlauf variiert, um auf diese Weise eine variierende Kante insbesondere am Fuß bzw. Kopf des Hygieneartikelverschlussbandes zu erzeugen.

Das Halbzeugbandmaterial kann unterschiedlich bevorratetet bzw. ausgeliefert werden. So ist es denkbar, das Halbzeugbandmaterial lediglich in Schlaufen lagenweise abgelegt zu bevorraten oder auszuliefern, die vorzugsweise in irgendwelche Behältnisse gelegt sind. Vorzugsweise wird jedoch das Halbzeugbandmaterial in Rollen oder Spulen bzw. Wicklungen bereitgestellt. Insbesondere Kreuzwicklungen haben sich, besonders bei einnutzigen Halbzeugbandmaterialien, als besonders geeignet erwiesen, da diese verhältnismäßig große Gebinde erlauben und verhältnismäßig stabile Spulen bzw. Wicklungen bereitstellen.

Insbesondere wenn Rollen, Spulen oder Wicklungen vorgesehen sind, kann es von Vorteil sein, die Rückseite des Trägerbandes in geeigneter Weise zu modifizieren, um einerseits stabile Rollen, Spulen oder Wicklungen zu erhalten und andererseits ein zu festes Anbacken der jeweils übereinander gewickelten Lagen untereinander zu vermeiden. Zwar kann ggf. hier eine Zwischenlage, wie ein ergänzender Bestandteil des Hygieneartikelverschlussbands, beispielweise ein Releaseband, oder ein durch ein Umfalten des Hygieneartikelverschlussbandes bzw. des Halbzeugbandmaterials gebildeter Bereich, oder sogar eine separate verlorene Zwischenfolie vorgesehen sein, was jedoch verhältnismäßig aufwendig ist. Dementsprechend ist es vorteilhaft, wenn die Rückseite des Trägerbandmittels zur Senkung und/oder Erhöhung der Haftreibung aufweist. Hierdurch lässt sich gezielt die Kontaktkraft zwischen den jeweiligen Lagen einstellen. Insbesondere eignet sich beispielsweise eine Silikonisierung, um derartige Mittel zur Senkung und/oder Erhöhung der Haftreibung bereitzustellen. Auch Prägungen zumindest der Rückseite des Trägerbands oder auch des gesamten Trägerbands können dementsprechend genutzt werden.

Vorgenannte Prägungen können bei geeigneter Ausgestaltung auch die Haptik des Trägerbandes in geeigneter Weise beeinflussen, um beispielsweise eine seidigere Haptik oder auch eine rauere Haptik einem Benutzer zu vermitteln. Gegebenenfalls können auch an der Vorderseite des Trägerbands gezielt Prägungen vorgesehen sein, um den Kontakt mit dem Klebstoff zu verbessern.

An der Vorderseite des Trägerbands können insbesondere zwei Klebstoffschichten bzw. Klebstoffbahnen vorgesehen sein, wobei eine erste Klebstoffschicht der beiden Klebstoffschichten bzw. eine erste Klebstoffbahn der beiden Klebstoffbahnen den ersten Klebefunktionsbereich und eine zweite Klebstoffschicht der beiden Klebstoffschichten bzw. eine zweite Klebstoffbahn der beiden Klebstoffbahnen den zweiten Klebefunktionsbereich bilden. Auf diese Weise lassen sich unterschiedliche peel-off-Kräfte konstruktiv besonders einfach bereitstellen.

Alternativ kann beispielsweise die Stärke der Klebstoffschichten bzw. Klebstoffbahnen variiert werden. Insbesondere kann es von Vorteil sein, die Stärke der Klebstoffschicht bzw. Klebstoffbahn im zweiten Klebefunktionsbereich dünner als im ersten Klebefunktionsbereich darzustellen, wodurch sich insbesondere bei verhältnismäßig dünnen Stärken der Klebstoffschichten bereits entsprechende Unterschiede in den peel-off-Kräften gewährleisten lassen. Gegebenenfalls kann auch eine Profilierung des Klebstoffs vorgesehen sein, um auf diese Weise die peel-off-Kräfte entsprechend zu beeinflussen. Hierbei wird in der Regel durch die Profilierung, wenn diese ausreichend stark gewählt ist, die peel-off-Kraft reduziert werden, sodass eine entsprechende Profilierung vorzugsweise im zweiten Klebefunktionsbereich vorgesehen ist. Es sind jedoch auch Klebstoffe denkbar, bei denen durch eine Profilierung beispielsweise der Wiederstand gegen shear-Kräfte erhöht werden können, sodass eine entsprechende Profilierung gegebenenfalls auch im ersten Klebefunktionsbereich dementsprechend vorteilhaft sein kann.

Vorzugsweise beträgt die Profiltiefe zumindest die Hälfte der Stärke des Klebstoffs, um auf einen ausreichend betriebssicheren Effekt bauen zu können.

Auch kann die Profilierung wenigstens ein Profiltal mit einem minimalen Taldurchmesser von wenigstens der Stärke des Klebstoffs bzw. von wenigstens 0,5 mm aufweisen, um auf diese Weise die Klebstoffeigenschaften nennenswert beeinflussen zu können.

Sind die beiden Klebstoffschichten vorzugsweise unmittelbar oder gar überlappend benachbart angeordnet, so können insbesondere minimale Abmessungen des entsprechenden Hygieneartikelverschlussbands gewährleistet werden.

Insbesondere können der erste Klebefunktionsbereich und der zweite Klebefunktionsbereich nicht mehr als 1 mm voneinander beabstandet ausgebildet sein. Bei einem derartig geringen Abstand ist zwischen den beiden Klebefunktionsbereichen die Ausbildung eines weiteren funktionalen Bereichs, wie beispielsweise eines Zwischenbereichs, welcher eine Elastizität des Trägerbands ermöglicht, ausgeschlossen. Insbesondere gelangt man auch in dem Bereich einer betriebssicheren maschinellen Fertigung, wenn das Hygieneartikelverschlussband dann an dem Hygieneartikel befestigt werden soll. Genau hier setzt die vorstehend bereits beschriebene Positionierung des Hygieneartikelverschlussbands an dem Hygieneartikel an, nach welcher die beiden Klebefunktionsbereiche des Hygieneartikelverschlussbands gemeinsam mit einer der beiden Verschlusszonen verbindbar bzw. verbunden sind. Hierbei ist es von Vorteil, wenn der erste Klebefunktionsbereich mit den höheren peel-off-Kräften zur Gänze und ein kleiner Teil des zweiten Klebeverschlussbereichs mit dem niedrigen peel-off-Kräften mit der ersten Verschlusszone verbunden sind, sodass sichergestellt ist, dass der erste Klebefunktionsbereich auch unter Berücksichtigung maschineller Ungenauigkeiten betriebssicher nie die zweite Verschlusszone erreicht. Auf diese Weise können Beschädigungen an der zweiten Verschlusszone durch den ersten Klebefunktionsbereich wirkungsvoll vermieden werden.

Insbesondere können die erste Verschlusszone und/oder die zweite Verschlusszone auf einer Folie mit einer Stärke von 25 µm oder kleiner bzw. mit einer Stärke dünner als das Trägerband vorgesehen sein. Die vorstehend erläuterten Hygieneartikelverschlussbänder können durch die Variationen der peel-off-Kräfte und die verhältnismäßig große Ausgestaltung des Klebefunktionsbereichs mit den geringeren peel-off-Kräften auch für sehr empfindliche Oberflächen, wie dieses beispielsweise Folien mit einer geringeren Stärke oder auch sehr empfindliche None-Woven darstellen, betriebssicher appliziert werden.

Wie bereits vorstehend im Detail erläutert, weist das Hygieneartikelverschlussband in der Regel eine Längserstreckungsrichtung auf und erstreckt sich entlang dieser längs. Insofern kann dem Hygieneartikelverschlussband auch ein Fuß an dem einen Abschluss des Hygieneartikelverschlussbands in Längserstreckungsrichtung und ein Kopf an dem anderen Abschluss des Hygieneartikelverschlussbandes in Längserstreckungsrichtung zugeordnet werden. Hierbei können der Fuß und der Kopf bzw. die entsprechenden Abschlüsse zum einen geradlinig aber auch gewellt, gebogen, spitz zulaufend oder ähnlich ausgestaltet sein. In der Regel werden der Fuß bzw. Kopf durch eine entsprechend ausgeformte Kante des Hygieneartikelverschlussbands gebildet. Zwischen dem Fuß und dem Kopf sind dann in der Regel weitere Kanten angeordnet, die in der Regel der durch den Trennvorgang gegebenen Form entsprechen.

Zumindest einer der beiden Klebefunktionsbereiche kann senkrecht zu der Längserstreckungsrichtung von einer Kante des Hygieneartikelverschlussbands zu einer weiteren Kante des Hygieneartikelverschlussbands reichen. Dieses entspricht beispielsweise einer Bereitstellung des Hygieneartikelverschlussbands aus einem Halbzeugbandmaterial mit entsprechend durchgängigen Klebstoffbahnen, die diese Klebefunktionsbereiche bereitstellen, wobei das Halbzeugbandmaterial senkrecht zur Banderstreckungsrichtung durchtrennt wird. Darüber hinaus gewährleistet diese Ausgestaltung eine ausreichende Breite des jeweiligen Klebefunktionsbereichs, sodass dieser seine Funktionalität entsprechend der Breite des Hygieneartikelverschlussbands entfalten kann. Vorzugsweise sind beide Klebefunktionsbereiche, insbesondere sogar alle Klebefunktionsbereiche, senkrecht zu der Längserstreckungsrichtung von der einen Kante des Hygieneartikelverschlussbands zu der weiteren Kante des Hygieneartikelverschlussbands ausgebildet. Dieses kann insbesondere für die anderen funktionalen Bereiche des Hygieneartikelverschlussbands gelten.

Auf besonders einfache Weise kann der erste Klebefunktionsbereich kleiner als der zweite Klebefunktionsbereich ausgebildet werden, wenn der erste Klebefunktionsbereich in Längserstreckungsrichtung schmaler als der zweite Klebefunktionsbereich ausgebildet ist.

Ein besonders kompaktes Hygieneartikelverschlussband ergibt sich, wenn dieses genau einen einzigen ersten Klebefunktionsbereich und genau einen einzigen zweiten Klebefunktionsbereich aufweist. Dieses gilt insbesondere wegen der völlig neuen Anordnung der beiden Klebefunktionsbereiche in unmittelbarer oder gar überlappend benachbarter Weise. Ebenso können bei einem entsprechend Halbzeugbandmaterial genau ein einziger erster Klebefunktionsbereich und genau ein einziger zweiter Klebefunktionsbereich vorgesehen sein, was letztlich jedoch lediglich bei einnutzigen Halbzeugbandmaterialien Sinn zu ergeben scheint.

Andererseits versteht es sich, dass das Halbzeugbandmaterial, wie bereits vorstehend erläutert, mehrnutzig ausgestaltet sein kann. Dann ist es vorteilhaft, wenn eine einen ersten Klebefunktionsbereich der beiden Klebefunktionsbereiche bildende Klebstoffbahn zwischen zwei den jeweils anderen Klebefunktionsbereich der beiden Klebefunktionsbereiche bildenden Klebstoffbahnen angeordnet ist. Hier kann dann das Halbzeugbandmaterial in der Klebstoffbahn bzw. in dem Klebefunktionsbereich, der zwischen den beiden anderen Klebstoffbahnen bzw. Klebefunktionsbereichen angeordnet ist, entlang der Banderstreckungsrichtung bzw. entlang der Maschinenrichtung getrennt werden, um auf diese Weise entsprechend zwei Hygieneartikelverschlussbänder, die zwar Kopf an Kopf oder Fuß an Fuß angeordnet sind, zu erhalten.

Es versteht sich, dass beidseits der beiden, den anderen Klebefunktionsbereich der beiden Klebefunktionsbereiche bildenden Klebstoffbahnen eine Bahn zur Bildung eines weiteren funktionalen Bereichs des Hygieneartikelverschlussbands, wie beispielsweise eine Bahn zur Bildung eines oder zweier Fingerlifte angeordnet sein kann.

Es versteht sich, dass jeder Klebstoff naturgemäß gewissen Schwankungen unterliegt, solange nur eine entsprechende Betrachtung mit genügend hoher Auflösung stattfindet. In vorliegendem Zusammenhang wird ein Klebstoff jedoch hinsichtlich seiner makroskopischen Eigenschaften beurteilt, sodass entsprechende mikroskopische oder mesoskopische Schwankungen unkritisch erscheinen. Insbesondere aus der EP 2 072 595 A1, der EP 2 431 435 A2 und der WO 2012/129428 A1 ergibt sich, dass Streifenbreiten von Klebstoff, die unter 4,8 mm liegen und abwechselnd angeordnet sind, bereits zu einem ausreichend konstanten flächigen Klebstoffverhalten führen, wie es für Hygieneartikelverschlussbänder gefordert ist.

Dementsprechend kann insbesondere ein Klebstoff eines Klebefunktionsbereichs als konstant angesehen sein, wenn dieser entsprechende unterschiedliche Klebstoffarten vereinigt, wie diese in dem vorgenannten Stand der Technik offenbart sind. Auch ist es denkbar, dass der Klebstoff wenigstens eines der beiden Klebefunktionsbereiche profiliert ist, wie bereits vorstehend erläutert. Dieses kann, je nach gewünschter Funktionalität des entsprechenden Klebfunktionsbereichs und des tatsächlich gewählten Klebstoffs, die Wirkung des jeweiligen Klebstoffs verstärken oder vermindern. Ersteres gilt insbesondere für Klebstoffe und Klebefunktionsbereiche, die in hinsichtlich ihr shear-Eigenschaften optimiert sein sollen, während letzteres häufig hinsichtlich der peel-off-Kräfte zu finden ist. Hierbei versteht es sich, dass, wenn die Profilierung ein Profiltal bzw. mehrere Profiltäler aufweist, diese in zumindest einer Richtung, vorzugsweise in Längserstreckungsrichtung des Hygieneartikelverschlussbands, einen Taldurchmesser unter 4,8 mm aufweisen sollen, um nach Möglichkeit konstante Klebstoffeigenschaften über den jeweiligen Klebefunktionsbereich bereit zu stellen.

Damit die Profilierung ihre Wirkung in ausreichendem Maße entfalten kann, ist es von Vorteil, wenn wenigstens ein Profiltal einen minimalen Taldurchmesser von wenigstens der Stärke des Klebstoffs bzw. von wenigstens 0,5mm aufweist. Bei kleineren Strukturen haben sich keine nennenswerten Verbesserungen hinsichtlich der shear-Eigenschaften feststellen lassen. Durch eine derartig kleine Profilierung lassen sich jedoch ggf. auch die peel-off-Eigenschaften beeinflussen, was ggf. auch mit sehr kleinen Profilierungen bereits zu nennenswerten Ergebnissen führt.

Vorzugsweise sind die peel-off-Kräfte des ersten Klebefunktionsbereichs zweimal, vorzugsweise zweieinhalbmal, größer als die peel-off-Kräfte des zweiten Klebefunktionsbereichs.

Da bei der vorstehenden Angabe hinsichtlich der peel-off-Kräfte letztlich Relativwerte zwischen den beiden Klebefunktionsbereichen verglichen werden, spielt die genaue Definition bzw. Wahl der Prüfung, mit welcher die peel-off-Kräfte gemessen werden, eine nur untergeordnete Rolle. Insbesondere bewährt hat sich eine Modifizierung der FENAT-Testmethode Nr. 9 (FTM 9; FENAT Technisches Handbuch, 6. Ausgabe 2001), welche auf einer Stahlplatte, insbesondere auf einer AFERA-Stahlplatte, durchgeführt wird. Hierzu wird vorzugsweise ein 15 mm (bei Abweichung gut kennzeichnen) breiter Teststreifen in Längsrichtung des Hygieneartikelverschlussbands mittels eines 2 kg Handüberrollers mit einer Belastung von 500 gr und einer Überrollgeschwindigkeit von 300 mm/min vor und zurück auf der Stahlplatte aufgetragen. Ein Ende des Prüflings, also dessen Kopf, wird mit einem durch ein Zwischenband überbrückten Abstand von 125mm mit einem Zugprüfgerät verbunden, um den Prüfling dann senkrecht zu der Stahlplatte mittels des Zugprüfgerätes bis zu seinem Fuß komplett abzuziehen. Die hierbei auftretenden Kräfte werden in Abhängigkeit von der Abzugsstrecke aufgetragen. Vorzugsweise erfolgt die Prüfung nach einer Klimatisierung auf 23 ± 2 °C und 50 ± 5 % relativer Luftfeuchtigkeit. Rollenware sollte mindestens 24 Stunden bzw. ein einzelnes Muster mindestens 4 Stunden klimatisiert werden. Angesichts mancher überprüfter Klebstoffe kann die Messung ggf. nicht mehrfach durchgeführt werden, da beispielsweise Klebstoffe mit hohen peel-off-Kräften ihre Eigenschaften nach einem ersten Abziehen zu sehr verändern und auch hierfür nicht ausgelegt sind. Stattdessen werden entsprechend dann mehrere Prüflinge einer derartigen Messung unterzogen, wobei es naturgemäß auf Grund der leichten Winkellage, der manuellen Positionierungen und auch gewisser Fertigungstoleranzen dazu kommt, dass der Wechsel zwischen den beiden Klebefunktionsbereichen an unterschiedlichen Positionen gemessen wird. Dieses ändert jedoch nicht den relativen Unterschied in den peel-off-Kräften an sich, der das Ziel dieser Messung ist.

Alternativ können statt der Stahlplatte auch andere Materialien, wie beispielsweise Glas, oder auch die für die Verschlusszonen gewählten Materialien des zu verschließenden Hygieneartikels, welche vorzugsweise auf einen festen Untergrund appliziert sein sollten, genutzt werden.

Insofern ist es von Vorteil, wenn das Hygieneartikelverschlussband an seinem Fuß den ersten Klebefunktionsbereich und dann anschließend in Richtung Kopf den zweiten Klebefunktionsbereich aufweist. Bei einer derartigen Ausgestaltung kann dann der zweite der beiden Klebefunktionsbereiche beispielsweise für einen öffenbaren bzw. wiederverschließbaren Bereich, insbesondere für einen Benutzerbereich, des Hygieneartikelverschlussbands genutzt werden, während der erste Klebefunktionsbereich mit den höheren peel-off-Kräften beispielsweise als entsprechender Permanentbereich, der permanent mit einer entsprechenden Verschlusszone verbunden werden soll, genutzt werden kann.

Wenn die beiden Klebefunktionsbereiche in unmittelbarer Nähe zueinander angeordnet sind, wird vorzugsweise ein Teil des zweiten Klebefunktionsbereich noch mit in der Verschlusszone des Hygieneartikels, in welcher das Hygieneartikelverschlussband permanent befestigt werden soll, befestigt wird, um Fertigungstoleranzen betriebssicher ausgleichen zu können und zu verhindern, dass der erste Klebefunktionsbereich mit der zweiten Verschlusszone, die mit dem Benutzerbereich bzw. mit dem öffenbaren und wiederverschließbaren Bereich in Kontakt kommen soll, in Kontakt kommt und dort ebenfalls permanent verklebt.

Vorzugsweise ist der erste Klebfunktionsbereich nicht breiter als 10 mm in Längserstreckungsrichtung des Hygieneartikelverschlussbands. Dieses spricht dem Erfordernis, das Hygieneartikelverschlussband mit möglichst geringen Abmessungen zu fertigen, sodass dementsprechend auch ein Klebstoff für den ersten Klebefunktionsbereich gewählt werden sollte, der diesen Abmessungen entsprechend Rechnung trägt und ausreichend hohe peel-off-Kräfte bereitstellt.

Insbesondere sollte der erste Klebefunktionsbereich 2,5 mm breit oder breiter in Längserstreckungsrichtung ausgebildet sein, um ausreichend betriebssicher bereitgestellt und an dem Hygieneartikel befestigt werden zu können. Zwar liegt eine Breite von 2,5 mm unter den 4,8 mm, welche nach dem Stand der Technik bereits als ausreichend schmal angesehen werden, um konstante Klebefunktionsbereiche an durch Klebstoffstreifen, die abwechselnd angeordnet sind, bereitstellen zu können. Da jedoch der erste Klebefunktionsbereich für sich alleine steht und lediglich von einem anderen Klebstoff benachbart ist, können sich seine hohen peel-off-Kräfte in ausreichendem Maße durchsetzten und dementsprechend das Verhalten des ersten Klebefunktionsbereichs bestimmen, auch wenn dieser derartig schmal gewählt ist.

Entsprechend einer Breite des Klebefunktionsbereich zwischen 2,5 und 10 mm ist es von Vorteil, wenn bei einem mehrnutzigen Halbzeugbandmaterial die den ersten Klebefunktionsbereich bildende Klebstoffbahn in Längserstreckung zwischen 5 mm und 20 mm erstreckt ist, sodass durch ein Trennen entlang der Maschinenrichtung bzw. senkrecht zur Längserstreckungsrichtung entsprechend breite Klebefunktionsbereiche des Hygieneartikelverschlussbands betriebssicher bereitgestellt werden können.

Dementsprechend ist es von Vorteil, wenn die den ersten Klebefunktionsbereich bildende Klebstoffbahn sich in Längserstreckungsrichtung nicht mehr als 20 mm und/oder 5 mm und mehr erstreckt.

Insbesondere kann der zweite Klebefunktionsbereich zwischen 10 mm und 30 mm breit sein, wodurch sichergestellt werden kann, dass der zweite Klebefunktionsbereich auch bei einer maschinellen Befestigung ausreichend fest positioniert verbleibt, ohne das die peel-off-Kräfte für dessen Klebstoff zu hoch gewählt werden müssen.

Dementsprechend ist es von Vorteil, wenn die Gesamtlänge des Klebeverschlussbands zwischen 2,5 mm und 40 mm beträgt, wobei ggf. noch kleinere Ansätze, wie beispielsweise Bereiche für einen Fingerlift oder ähnliches ergänzend vorgesehen sein können.

Das Breitenverhältnis zwischen dem ersten und dem zweiten Klebefunktionsbereich liegt vorzugsweise zwischen 1:2 und 1:5 in Längserstreckung des Hygieneartikelverschlussbands gesehen. Durch ein derartiges Breitenverhältnis kann der unterschiedlichen Ausprägung der peel-off-Kräfte in den beiden Klebefunktionsbereichen gut Rechnung getragen werden und ein möglichst gleichmäßiges Anheften während der maschinellen Befestigung des Hygieneartikelverschlussbands an dem jeweiligen Hygieneartikel gewährleistet werden.

Es versteht sich, dass die Merkmale der vorstehend bzw. in den Ansprüchen beschriebenen Lösungen gegebenenfalls auch kombiniert werden können, um die Vorteile entsprechend kumuliert umsetzen zu können.

Weitere Vorteile, Ziele und Eigenschaften vorliegender Erfindung werden anhand nachfolgender Beschreibung von Ausführungsbeispielen erläutert, die insbesondere auch in anliegender Zeichnung dargestellt sind. In der Zeichnung zeigen:
- Figur 1: ein Hygieneartikelverschlussband in einer Seitenansicht;
- Figur 2: das Hygieneartikelverschlussband nach Figur 1 in einer Ansicht auf die Vorderseite, wobei Figuren b und c Alternativen des in Figuren 1 und 2a dargestellten Hygieneartikelverschlussbands darstellen;
- Figur 3: das Hygieneartikelverschlussband nach Figuren 1 und 2 in einer Applikation an einer Bindenverpackung;
- Figur 4: ein mögliches Halbzeug zur Herstellung des Hygieneartikelverschlussbandes nach Figuren 1 bis 3;
- Figur 5: ein mögliches Halbzeug zur Herstellung des Hygieneartikelverschlussbands nach Figur 2c;

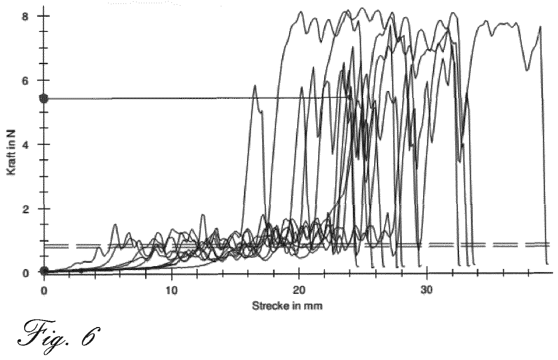

- Figur 6: ein Messprotokoll zur Messung von peel-off-Kräften.

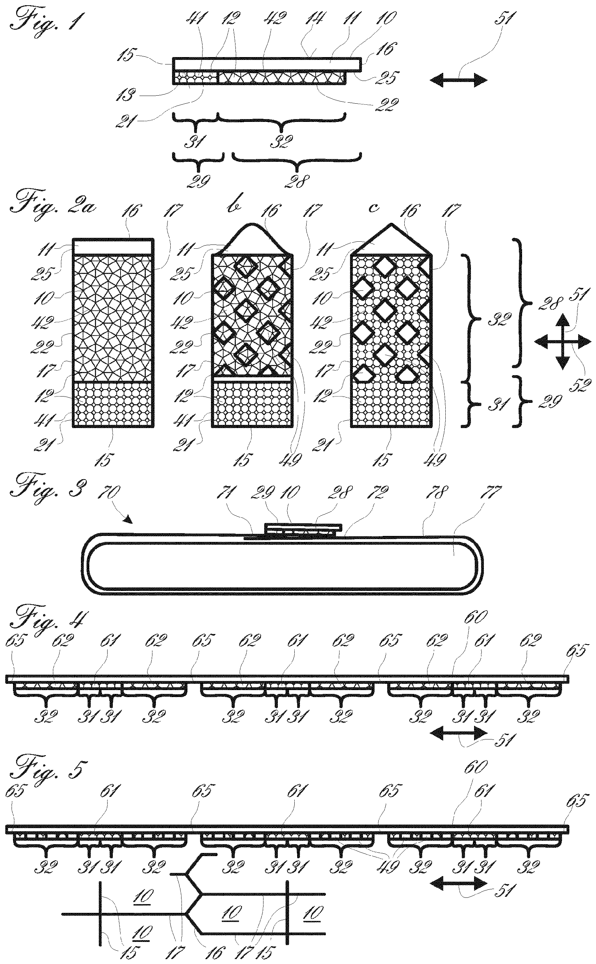

Das in den Figuren 1 und 2 dargestellte Hygieneartikelverschlussband 10 umfasst ein Trägerband 11, welches auf seiner Vorderseite 13 Klebstoff 12 trägt und eine Rückseite 14 aufweist.

Hierbei trägt das Trägerband 11 im Einzelnen eine erste Klebstoffschicht 41 und eine zweite Klebstoffschicht 42, wobei der jeweilige Klebstoff 12 der Klebstoffschicht 41, 42 sich in seinen peel-off-Kräften dahingehend unterscheidet, dass die peel-off-Kraft des Klebstoffs 12 der ersten Klebstoffschicht 41 mehr als doppelt so hoch ist, wie die peel-off-Kraft des Klebstoffs 12 der zweiten Klebstoffschicht 42.

Hierbei sind nach dem Ausführungsbeispielen der Figur 2c lediglich eine durchgehende Klebstoffschicht 41 vorgesehen, die eine Profilierung aufweist, wobei Ausnehmungen 49 vorgesehen sind, die bis zu dem Trägerband 11 durchreichen, sodass die Profiltiefe der Stärke der Klebstoffschicht 41 bzw. der Klebstoffbahn 61 (siehe Figur 5) entspricht. Bei dem in Figur 2b dargestellten Ausführungsbeispiel sind die Ausnehmungen 49 nicht so tief, sondern reichen lediglich bis zur Hälfte, so dass die Stärke der Klebstoffschicht 41 bzw. der Klebstoffbahn 61 im Bereich der Ausnehmungen 49 lediglich halb so groß ist, wie die Stärke der Klebstoffschicht 61 bzw. der Klebstoffbahn 61 im Übrigen, was immer noch bei dem verwendeten Klebstoff 12 ausreichend ist, um die peel-off-Kräfte des Klebstoffs 12 in diesem Bereich ausreichend herabzusenken.

Die Ausnehmungen 49 sind bei den in Figuren 2b und 2c sowie 5 dargestellten Ausführungsbeispielen mit einer Talbreite von ungefähr 2 mm ausgestattet.

Bei vorliegenden Ausführungsbeispielen wurden die peel-off-Kräfte entsprechend der vorstehend aufgeführten Prüfvorschrift gemessen, wobei Figur 10 ein entsprechendes Messprotokoll für verschiedene Hygieneartikelverschlussbänder 10 mit identischer Ausgestaltung darstellt. Die Unterschiede in den einzelnen Messkurven, bei denen die hohen peel-off-Kräfte und mithin die erste Klebstoffschicht 41 auftreten, liegen in den naturgemäßen Messungenauigkeiten des Messverfahrens, wobei das vorliegende Hygieneartikelverschlussband 10 jeweils einen Fuß 15 und einen Kopf 16 aufweist, der Kopf 16 auf Seiten der zweiten Klebstoffschicht 42 und der Fuß 15 auf Seiten der ersten Klebstoffschicht 41 vorgesehen ist und das Hygieneartikelverschlussband 10 vom Kopf 16 zum Fuß 15 hin jeweils abgezogen wurde, um die Messergebnisse der Figur 10 zu erhalten.

Zwischen den Kanten, welche den Fuß 15 und den Kopf 16 bilden, sind darüber hinaus Kanten 17 vorgesehen, welche einander gegenüberliegen und parallel zu einer Längserstreckungsrichtung 51, die sich vom Fuß 15 zu dem Kopf 16 erstreckt, ausgerichtet sind. Es versteht sich, dass in abweichenden Ausführungsformen die Kanten des Fußes 15 oder des Kopfes 16 bzw. die Kanten 17 nicht zwingend geradlinig ausgebildet sein müssen, wie beispielhaft an Hand der Figuren 2b und 2c dargestellt ist.

Senkrecht zu der Längserstreckungsrichtung 51 kann eine Breite des Hygieneartikelverschlussbands 10 definiert werden, welche von einer Kante 17 zu der anderen Kante 17 reicht und parallel zu einer Banderstreckungsrichtung 52 verläuft, welche die Erstreckung eines Halbzeugbandmaterials bezeichnet, von welchem das Hygieneartikelverschlussband 10 durch eine Trennung entlang der Kanten 17 hergestellt werden kann.

Die erste Klebstoffschicht 41 und die zweite Klebstoffschicht 42 der Ausführungsbeispiele nach Figuren 2a und 2b bzw. der nicht profilierte Bereich und der profilierte Bereich der Klebstoffschicht 41 des Ausführungsbeispiels nach Figur 2c bilden jeweils einen ersten Klebefunktionsbereich 31 und einen zweiten Klebefunktionsbereich 32 mit unterschiedlichen peel-off-Kräften, wie vorliegend erläutert. Darüber hinaus liegen die beiden Klebefunktionsbereiche 31, 32 bzw. die zugehörigen Klebstoffschichten 41, 42 unmittelbar nebeneinander, wobei ein etwaiger Abstand oder Überlappen lediglich durch Produktionsungenauigkeiten bedingt ist und insbesondere kein weiterer funktionaler Bereich des Hygieneartikelverschlussbands 10 hierzwischen vorgesehen ist oder vorgesehen werden kann. Maximal soll hier ein Abstand von 1 mm vorgesehen sein, wie beispielhaft anhand der Figur 2b dargestellt.

Dementsprechend bilden die beiden Klebefunktionsbereiche 31, 32 erste und zweite funktionale Bereiche 21, 22, von denen der erste funktionale Bereich 21 wesentlich höhere peel-off-Kräfte aufweist als der zweite funktionale Bereich 22.

Darüber hinaus umfasst das Hygieneartikelverschlussband 10 noch einen weiteren funktionalen Bereich 25, der als Fingerlift dient. Dieser wird bei vorliegendem Ausführungsbeispiel dadurch bereitgestellt, dass ein Bereich des Trägerbands 11 nicht mit einem Klebstoff 12 beschichtet ist.

Es versteht sich, dass in abweichenden Ausführungsformen, die insbesondere auch möglicherweise andere Funktionalitäten bereitstellen sollen, weitere funktionale Bereiche oder auch weitere Klebefunktionsbereiche vorgesehen sein können.

Durch den ersten funktionalen Bereich 21 wird ein Permanentbereich 29 des Hygieneartikelverschlussbands 10 bereitgestellt, welcher dazu dient, das Hygieneartikelverschlussband 10 an einem Hygieneartikel 70 (siehe Figur 3) permanent zu befestigen. Ein wenig von dem Permanentbereich 29 in Richtung des Kopfes 16 entfernt, lässt sich ein Benutzerbereich 28 definieren, in welchem der zweite funktionale Bereich 22 und der den Fingerlift bildende funktionale Bereich 25 angeordnet sind. Der Benutzerbereich 28 dient dazu, dass ein Benutzer den durch das Hygieneartikelverschlussband 10 gebildeten Verschluss ggf. öffnen und verschließen kann.

Der geringe Abstand, der zwischen dem Benutzerbereich 28 und dem Permanentbereich 29 vorgesehen ist, dient dazu, etwaige Toleranzen bei der Applikation des Hygieneartikelverschlussbands 10 an dem Hygieneartikel 70 aufzufangen, indem auch ein schmaler Bereich des zweiten funktionalen Bereichs 22 an einer ersten Verschlusszone 71 des Hygieneartikels 70 befestigt wird, die dazu dient, eine permanente Befestigung mit dem Hygieneartikelverschlussband 10 einzugehen.

Am Beispiel einer Umverpackung 78 für eine Damenbinde 77 kann nachvollzogen werden, dass ein entsprechender Verschluss der Umverpackung dadurch gewährleistet ist, dass die Umverpackung 78 bzw. der Hygieneartikel 70 die erste Verschlusszone 71 und eine zweite Verschlusszone 72 aufweist, wobei das Hygieneartikelverschlussband 10 die beiden Verschlusszonen 71, 72 überbrückt.

Auf Grund der hohen peel-off-Kräfte des ersten funktionalen Bereichs 21 ist das Hygieneartikelverschlussband 10 an der ersten Verschlusszone 51 der Umverpackung 78 bzw. des Hygieneartikels 70 permanent befestigt, während die geringeren peel-off-Kräfte des zweiten funktionalen Bereichs 22 eine Öffen- und Schließbarkeit durch einen Benutzer ermöglichen, wozu insbesondere auch der funktionale Bereich 25 als Fingerlift beiträgt.

Wie unmittelbar an Hand der Figur 2 ersichtlich, kann das Hygieneartikelverschlussband 10 durch Trennung parallel zur Längserstreckungsrichtung 51 von einem Halbzeugbandmaterial, welches entsprechend aufgebaut ist und sich entlang der Banderstreckungsrichtung 52 erstreckt, bereitgestellt werden.

Besonders bevorzugt wird ein mehrnutziges Halbzeugbandmaterial 60, wie es exemplarisch in Figur 4 für das in Figur 2a dargestellte Hygieneartikelverschlussband 10 und in Figur 5 für das in Figur 2c dargestellte Hygieneartikelverschlussband 10 dargestellt ist, genutzt.

Zum Bereitstellen des ersten Klebefunktionsbereichs 31 und mithin des ersten funktionalen Bereichs 21 trägt das in Figur 4 dargestellte Halbzeugbandmaterial 60 eine erste Klebstoffbahn 61, die beidseits von zweiten Klebstoffbahnen 62 begrenzt ist, welche jeweils der Bereitstellung eines zweiten Klebefunktionsbereichs 32 und mithin von zweiten funktionalen Bereichen 22 dienen. Bei dem in Figur 5 dargestellten Ausführungsbeispiel trägt das Halbzeugbandmaterial 60 zum Bereitstellen beider Klebefunktionsbereiche 31, 32 und mithin beider funktionaler Bereiche 21, 22 lediglich eine Klebstoffbahn 61, die jedoch in ihrem äußeren Bereich eine entsprechende Profilierung mit Ausnehmungen 49 aufweist, sodass jeweils der erste Klebefunktionsbereich 31 bzw. der erste funktionale Bereich 21 durch den zentralen nichtprofilierten Bereich der Klebstoffbahn 61 und jeweils zwei zweite Klebefunktionsbereiche 32 und mithin zwei funktionale Bereiche 22 durch die profilierten Bereiche der Klebstoffbahn 61 bereitgestellt werden können.

Bei dem in Figur 4 dargestellten Ausführungsbeispiel sind die zweiten Klebstoffbahnen 62 auf ihrer der jeweils ersten Klebstoffbahn 61 abgewandten Seite von einer klebstofffreien Bahn 65 begrenzt, welche der Ausgestaltung des funktionalen Bereichs 25 zur Bildung des Fingerlifts dienen.

Dementsprechend ist die Klebstoffbahn 61 des Ausführungsbeispiels nach Figur 5 beidseits von einer ldebstofffreien Bahn 65 begrenzt, welche der Ausgestaltung des funktionalen Bereichs 25 zur Bildung des Fingerlifts dienen. Um hierbei die Ausgestaltung der Kante, welche den Kopf 16 bildet, zu ermöglichen, ist der Schnitt durch die klebstofffreie Bahn 65 entsprechend variiert, wie in Figur 5 exemplarisch und schematisch dargestellt, wobei auch die Trennung parallel zur Längserstreckungsrichtung 51 zur Bereitstellung der Kanten 17 entsprechend versetzt erfolgt, wodurch ohne Verschnitt bzw. bei minimalem Verschnitt ein entsprechend ausgeformter Fingerlift bereitgestellt werden kann.

Insofern werden, um aus dem Halbzeugbandmaterial 60 Hygieneartikelverschlussbänder 10 bereitzustellen, die in Figuren 4 und 5 schematisch dargestellten Anordnungen nicht nur senkrecht zu ihrer Banderstreckungsrichtung 52 bzw. in Längserstreckungsrichtung 51 sondern auch parallel zur Banderstreckungsrichtung 52 durchtrennt, wobei unmittelbar nach dem Durchtrennen zunächst Kopf 16 an Kopf 16 bzw. Fuß 15 an Fuß 15 angeordnete Hygieneartikelverschlussbänder 10 bereitgestellt werden.

Diese Vorgehensweise ermöglicht es insbesondere, dass die notwendigen Toleranzen, mit denen die Klebstoffbahnen 61, 62 aufgebracht werden müssen, etwas geringer sind, da entsprechende Trenneinrichtungen jeweils in der Klebstoffbahn 61 bzw. in der Bahn 65 laufen und mithin kleinere Schwankungen tolerierbar erscheinen.

An der Rückseite 14 kann je nach konkreten Erfordernissen eine Prägung oder eine Silikonisierung vorgesehen sein, um das Verhalten des Hygieneartikelverschlussbandes 10 bzw. eines entsprechenden Halbzeugbandmaterials 60, welches zu dessen Herstellung genutzt wird, in geeigneter Weise zu beeinflussen, wenn dieses aufgerollt, aufgewickelt oder aufgespult werden soll. Sollte das Trägerband 11 in seinen Eigenschaften ausreichend friedlich mit den Klebstoffen 12 wechselwirken, kann auf derartige Maßnahmen auch verzichtet werden.

Auch können derartige Maßnahmen dazu dienen, die Haptik des Trägerbands 11 in geeigneter Weise zu beeinflussen.

### Bezugszeichenliste:

- 10: Hygieneartikelverschlussband
- 11: Trägerband
- 12: Klebstoff (exemplarisch beziffert)
- 13: Vorderseite
- 14: Rückseite
- 15: Fuß
- 16: Kopf
- 17: Kante

- 21: erster funktionaler Bereich
- 22: zweiter funktionaler Bereich
- 25: funktionaler Bereich
- 28: Benutzerbereich
- 29: Permanentbereich

- 31: erster Klebefunktionsbereich
- 32: zweiter Klebefunktionsbereich
- 41: erste Klebstoffschicht
- 42: zweite Klebstoffschicht
- 49: Ausnehmung

- 51: Längserstreckungsrichtung
- 52: Banderstreckungsrichtung

- 60: Halbzeugbandmaterial
- 61: erste Klebstoffbahn
- 62: zweite Klebstoffbahn
- 65: Bahn

- 70: Hygieneartikel
- 71: erste Verschlusszone
- 72: zweite Verschlusszone
- 77: Damenbinde
- 78: Umverpackung

## Patentansprüche

1. Klebendes Hygieneartikelverschlussband (10) mit einem Trägerband (11), welches eine Klebstoff (12) tragende Vorderseite (13) und eine Rückseite (14) aufweist, wobei an der Vorderseite (13) wenigstens eine Klebstoffschicht (41, 42), die einen ersten Klebefunktionsbereich (31) und einen zweiten Klebefunktionsbereich (32) bildet, vorgesehen ist, **dadurch gekennzeichnet, dass** peel-off-Kräfte des ersten Klebefunktionsbereichs (31) der beiden Klebefunktionsbereiche (31, 32) größer als peel-off-Kräfte des zweiten Klebefunktionsbereichs (32) der beiden Klebefunktionsbereiche (31, 32) sind, wobei der erste Klebefunktionsbereich (31) der beiden Klebefunktionsbereiche (31, 32) einen Permanentbereich bildet, wobei der Permanentbereich (29) dazu dient, das Hygieneartikelverschlussband (10) an einem Hygieneartikel (70) permanent zu befestigen, und der zweite Klebefunktionsbereich (32) der beiden Klebefunktionsbereiche (31, 32) einen Benutzerbereich bildet, wobei der Benutzerbereich (28) dazu dient, dass ein Benutzer den durch das Hygieneartikelverschlussband (10) gebildeten Verschluss öffnen und verschließen kann, und dass der erste Klebefunktionsbereich (31) kleiner als der zweite Klebefunktionsbereich (32) ausgebildet ist

2. Hygieneartikelverschlussband (10) nach Anspruch 1, **dadurch gekennzeichnet, dass** an der Vorderseite (13) zwei Klebstoffschichten (41, 42) vorgesehen sind, wobei eine erste Klebstoffschicht (41) der beiden Klebstoffschichten (41, 42) den ersten Klebefunktionsbereich (31) und eine zweite Klebstoffschicht (42) der beiden Klebstoffschichten (41, 42) den zweiten Klebefunktionsbereich (32) bilden, wobei die beiden Klebstoffschichten (41, 42) vorzugsweise unmittelbar benachbart oder gar überlappend t angeordnet sind.

3. Hygieneartikelverschlussband (10) nach Anspruch 1 oder 2, **dadurch gekennzeichnet, dass** sich das Hygieneartikelverschlussband (10) von einem Fuß (15) bis zu einem Kopf (16) entlang einer Längserstreckungsrichtung (51) längs erstreckt.

4. Hygieneartikelverschlussband (10) nach Anspruch 3, **dadurch gekennzeichnet, dass** zumindest einer der beiden Klebefunktionsbereiche (31, 32) senkrecht zu der Längserstreckungsrichtung (51) von einer Kante (17) des Hygieneartikelverschlussbands (10) zu einer weiteren Kante (17) des Hygieneartikelverschlussbands (10) reicht.

5. Hygieneartikelverschlussband (10) nach Anspruch 3 oder 4, **dadurch gekennzeichnet, dass** der erste Klebefunktionsbereich (31) in Längserstreckungsrichtung (51) schmaler als der zweite Klebefunktionsbereich (32) ausgebildet ist.

6. Halbzeugbandmaterial (60) zur Herstellung eines sich entlang einer senkrecht zu einer Banderstreckungsrichtung (52) des Halbzeugbandmaterials (60) ausgerichteten Längserstreckungsrichtung (51) längs erstreckenden klebenden Hygieneartikelverschlussbands (10), insbesondere nach einem der Ansprüche 1 bis 5, mit einem Trägerband (11), welches eine Klebstoff (12) tragende Vorderseite (13) und eine Rückseite 14) aufweist, wobei an der Vorderseite (13) wenigsten eine Klebstoffbahn (61, 62), die einen ersten Klebefunktionsbereich (31) und einen zweiten Klebefunktionsbereich (32) bildet, vorgesehen ist, **dadurch gekennzeichnet, dass** peel-off-Kräfte des ersten Klebefunktionsbereichs (31) der beiden Klebefunktionsbereiche (31, 32) größer als peel-off-Kräfte des zweiten Klebefunktionsbereichs (32) der beiden Klebefunktionsbereiche (31, 32) sind, wobei der erste Klebefunktionsbereich (31) der beiden Klebefunktionsbereiche (31, 32) einen Permanentbereich bildet, wobei der Permanentbereich (29) dazu dient, das Hygieneartikelverschlussband (10) an einem Hygieneartikel (70) permanent zu befestigen, und der zweite Klebefunktionsbereich (32) der beiden Klebefunktionsbereiche (31, 32) einen Benutzerbereich bildet, wobei der Benutzerbereich (28) dazu dient, dass ein Benutzer den durch das Hygieneartikelverschlussband (10) gebildeten Verschluss öffnen und verschließen kann, und dass der erste Klebefunktionsbereich (31) in Längserstreckungsrichtung (51) schmaler als der zweite Klebefunktionsbereich (32) ausgebildet ist

7. Halbzeugbandmaterial (60) nach Anspruch 6, **dadurch gekennzeichnet, dass** an der Vorderseite (13) zwei Klebstoffbahnen (61, 62) vorgesehen sind, wobei eine erste Klebstoffbahn (61) der beiden Klebstoffbahnen (61, 62) den ersten Klebefunktionsbereich (31) und eine zweite Klebstoffbahn (62) der beiden Klebstoffbahnen (61, 62) den zweiten Klebefunktionsbereich (32) bilden, wobei die beiden Klebstoffschichten (41, 42) vorzugsweise unmittelbar oder gar überlappend benachbart angeordnet sind

8. Hygieneartikelverschlussband (10) nach einem der Ansprüche 1 bis 5 bzw. Halbzeugbandmaterial (60) nach Anspruch 6 oder 7, **dadurch gekennzeichnet, dass** die peel-off-Kräfte des ersten Klebefunktionsbereichs (31) doppelt, vorzugsweise zweieinhalb mal, größer als die peel-off-Kräfte des zweiten Klebefunktionsbereichs (32) sind.

9. Hygieneartikelverschlussband (10) nach einem der Ansprüche 1 bis 5 und 8 bzw. Halbzeugbandmaterial (60) nach einem der Ansprüche 6 bis 8, **gekennzeichnet durch** genau einen einzigen ersten Klebefunktionsbereich (31) und genau einen einzigen zweiten Klebefunktionsbereich (32).

10. Halbzeugbandmaterial (60) nach einem der Ansprüche 6 bis 8, **dadurch gekennzeichnet, dass** es mehrnutzig ausgestaltet ist und eine einen ersten Klebefunktionsbereich (31) bildende Klebstoffbahn (61) zwischen zwei zweite Klebefunktionsbereiche (32) bildenden Klebstoffbahnen (62) angeordnet ist.

11. Halbzeugbandmaterial (60) nach Anspruch 10, **dadurch gekennzeichnet, dass** beidseits der beiden, die zweiten Klebefunktionsbereiche (32) bildenden Klebstoffbahnen (62) jeweils eine Bahn (65) zur Bildung eines weiteren funktionalen Bereichs (25) des Hygieneartikelverschlussbands (10) angeordnet ist.

12. Halbzeugbandmaterial (60) nach Anspruch 10 oder 11, **dadurch gekennzeichnet, dass** die den ersten Klebefunktionsbereich (31) bildende Klebstoffbahn (61) sich in Längserstreckungsrichtung nicht mehr als 20 mm erstreckt.

13. Mit einem Hygieneartikelverschlussband (10) nach einem der Ansprüche 1 bis 5, 8 und 9 verschlossener oder verschließbarer Hygieneartikel (70), umfassend eine erste Verschlusszone (71) und eine zweite Verschlusszone (72), die miteinander zu verschließen sind, und das Hygieneartikelverschlussband (10), welches mit seinem einen Permanentbereich (29) bildenden ersten Klebefunktionsbereich (31) permanent mit der ersten Verschlusszone (71) und mit seinem einen Benutzerbereich (28) bildenden zweiten Klebefunktionsbereich (32) mit der zweiten Verschlusszone (72) verbunden, verbindbar oder lösbar verbunden ist, **dadurch gekennzeichnet, dass** die beiden Klebefunktionsbereiche (31, 32) des Hygieneartikelverschlussbands (10) gemeinsam mit der ersten Verschlusszone (71) verbindbar bzw. verbunden sind.

14. Hygieneartikel (70) nach Anspruch 13, **dadurch gekennzeichnet, dass** die erste Verschlusszone (71) und/oder die zweite Verschlusszone (72) auf eine Folie mit einer Stärke von 25 µm oder kleiner bzw. mit einer Stärke dünner als das Trägerband (11) vorgesehen ist.

15. Hygieneartikelverschlussband (10) nach einem der Ansprüche 1 bis 5, 8 und 9, Halbzeugbandmaterial (60) nach einem der Ansprüche 6 bis 12 bzw. Hygieneartikel (70) nach Anspruch 13 oder 14, **dadurch gekennzeichnet, dass** der erste Klebfunktionsbereich (31) und der zweite Klebefunktionsbereich (32) nicht mehr als 1 mm voneinander beabstandet sind.

16. Hygieneartikelverschlussband (10) nach einem der Ansprüche 1 bis 5, 8, 9 und 15, Halbzeugbandmaterial (60) nach einem der Ansprüche 6 bis 12 und 15 bzw. Hygieneartikel (70) nach Anspruch 13 oder 15, **dadurch gekennzeichnet, dass** der erste Klebefunktionsbereich (31) zwischen 2,5 und 10 mm breit und/oder der zweite Klebefunktionsbereich (32) zwischen 10 mm und 30 mm breit sind und/oder dass ein Breitenverhältnis zwischen dem ersten und dem zweiten Klebefunktionsbereich (31, 32) zwischen 1:2 und 1:5 liegt.

17. Hygieneartikelverschlussband (10) nach einem der Ansprüche 1 bis 5, 8, 9, 15 und 16, Halbzeugbandmaterial (60) nach einem der Ansprüche 6 bis 12, 15 und 16 bzw. Hygieneartikel (70) nach Anspruch 13 bis 16, **dadurch gekennzeichnet, dass** zumindest die Rückseite (14), vorzugsweise das gesamte Trägerband (11), eine Prägung aufweist.

18. Hygieneartikelverschlussband (10) nach einem der Ansprüche 1 bis 5, 8, 9 und 15 bis 17, Halbzeugbandmaterial (60) nach einem der Ansprüche 6 bis 12 und 15 bis 17 bzw. Hygieneartikel (70) nach Anspruch 13 bis 17, **dadurch gekennzeichnet, dass** der Klebstoff (12) wenigstens eines der beiden Klebefunktionsbereiche (31, 32) profiliert ist, wobei die Profilierung vorzugsweise eine Profiltiefe aufweist, die zumindest die Hälfte der Stärke des Klebstoff (12) beträgt und/oder wobei die Profilierung vorzugsweise wenigstens ein Profiltal mit einem minimalen Taldurchmesser von wenigstens der Stärke des Klebstoffs (12) bzw. von wenigstens 0,5 mm aufweist.

19. Hygieneartikelverschlussband (10) nach einem der Ansprüche 1 bis 5, 8, 9 und 15 bis 18, Halbzeugbandmaterial (60) nach einem der Ansprüche 6 bis 12 und 15 bis 18 bzw. Hygieneartikel (70) nach Anspruch 13 bis 18, **dadurch gekennzeichnet, dass** die Rückseite (14) Mittel zur Senkung und/oder Erhöhung der Haftreibung trägt und insbesondere silikonisiert ist.

## Claims

1. An adhesive hygiene item closure tape (10) with a carrier tape (11), which comprises a front side (13) carrying adhesive (12) and a rear side (14), wherein at least one adhesive layer (41, 42), which forms a first adhesive functional region (31) and a second adhesive functional region (32), is provided at the front side (13), **characterised in that** peel-forces of the first adhesive functional region (31) of the two adhesive functional regions (31, 32) are greater than peel-off forces of the second adhesive functional region (32) of the two adhesive functional regions (31, 32), wherein the first adhesive functional region (31) of the two adhesive functional regions (31, 32) forms a permanent region, wherein the permanent region (29) serves to permanently fasten the hygiene item closure tape (10) to a hygiene item (70), and the second adhesive functional region (32) of the two adhesive functional regions (31, 32) forms a user region, wherein the user region (28) permits a user to open and close the closure formed by the hygiene item closure tape (10), and that the first adhesive functional region (31) is constituted smaller than the second adhesive functional region (32).

2. The hygiene item closure tape (10) according to claim 1, **characterised in that** two adhesive layers (41, 42) are provided at the front side (13), wherein a first adhesive layer (41) of the two adhesive layers (41, 42) forms the first adhesive functional region (31) and the second adhesive layer (42) of the two adhesive layers (41, 42) forms the second adhesive functional region (32), wherein the two adhesive layers (41, 42) are preferably arranged directly adjacent or even overlapping.

3. The hygiene item closure tape (10) according to claim 1 or 2, **characterised in that** the hygiene item closure tape (10) extends from a foot (15) up to a head (16) along a Longitudinal direction (51).

4. The hygiene item closure tape (10) according to claim 3, **characterised in that** at least one of the two adhesive functional regions (31, 32) extends normal to the longitudinal direction (51) from an edge (17) of the hygiene item closure tape (10) to a further edge (17) of the hygiene item closure tape (10).

5. The hygiene item closure tape (10) according to claim 3 or 4, **characterised in that** the first adhesive functional region (31) is constituted narrower in the longitudinal direction (51) than in the second adhesive functional region (32).

6. A semi-finished tape material (60) for the production an adhesive hygiene item closure tape (10) extending along longitudinal direction (51) aligned normal to a tape extension direction (52) of the semi-finished tape material (60), in particular according to any one of claims 1 to 5, with a carrier tape (11), which comprises a front side (13) carrying adhesive (12) and a rear side (14), wherein at least one line of adhesive (61, 62), which forms a first adhesive functional region (31) and a second adhesive functional region (32), is provided at the front side (13), **characterised in that** peel-forces of the first adhesive functional region (31) of the two adhesive functional regions (31, 32) are greater than peel-off forces of the second adhesive functional region (32) of the two adhesive functional regions (31, 32), wherein the first adhesive functional region (31) of the two adhesive functional regions (31, 32) forms a permanent region, wherein the permanent region (29) serves to permanently fasten the hygiene item closure tape (10) to a hygiene item (70), and the second adhesive functional region (32) of the two adhesive functional regions (31, 32) forms a user region, wherein the user region (28) permits a user to open and close the closure formed by the hygiene item closure tape (10), and that the first adhesive functional region (31) is constituted narrower than the second adhesive functional region (32) in the longitudinal direction (51).

7. The semi-finished tape material (60) according to claim 6, **characterised in that** two lines of adhesive (61, 62) are provided on the front side (13), wherein a first line of adhesive (61) of the two lines of adhesive (61, 62) forms the first adhesive functional region (31) and a second line of adhesive (62) of the two lines of adhesive (61, 62) forms the second adhesive functional region (32), wherein the two adhesive layers (41, 42) are preferably arranged directly adjacent or even overlapping.

8. The hygiene item closure tape (10) according to any one of claims 1 to 5 or semi-finished tape material (60) according to claim 6 or 7, **characterised in that** the peel-off forces of the first adhesive functional region (31) are double, preferably two and a half times greater than the peel-off forces of the second adhesive functional region (32).

9. The hygiene item closure tape (10) according to any one of claims 1 to 5 and 8 or semi-finished tape material (60) according to any one of claims 6 to 8, **characterised by** precisely one single first adhesive functional region (31) and precisely one single second adhesive functional region (32).

10. The semi-finished tape material (60) according to any one claims 6 to 8, **characterised in that** it has a multi-use design and a line of adhesive (61) forming a first adhesive functional region (31) is arranged between two lines of adhesive (62) forming the second adhesive functional region (32).

11. The semi-finished tape material (60) according to claim 10, **characterised in that** a line (65) for forming a further functional region (25) of the hygiene item closure tape (10) is arranged respectively on both sides of the lines of adhesive (62) forming the second adhesive functional region (32).

12. The semi-finished tape material (60) according to claim 10 or 11, **characterised in that** the line of adhesive (61) forming the first adhesive functional region (31) extends not more than 20 mm in the longitudinal direction.

13. A hygiene item (70) which is closed or can be closed with a hygiene item closure tape (10) according to any one of claims 1 to 5, 8 and 9, comprising a first closure zone (71) and a second closure zone (72), which are to be closed with one another, and the hygiene item closure tape (10) which is or can be permanently connected with its first adhesive functional region (31) forming its permanent region (29) to the first closure zone (71) and is or can be connected or connected detachably with its second adhesive functional region (32) forming a user region (28) to the second closure zone (72), **characterised in that** both adhesive functional regions (31, 32) of the hygiene item closure tape (10) can be connected or are connected jointly to the first closure zone (71).

14. The hygiene item (70) according to claim 13, **characterised in that** the first closure zone (71) and/or the second closure zone (72) is provided on a film with a thickness of 25 µm or less or with a thickness thinner than the carrier tape (11).

15. The hygiene item closure tape (10) according to any one of claims 1 to 5, 8 and 9, the semi-finished tape material (60) according to any one of claims 6 to 12 or hygiene item (70) according to claim 13 or 14, **characterised in that** the first adhesive functional region (31) and the second adhesive functional region (32) are not spaced apart from one another by more than 1 mm.

16. The hygiene item closure tape (10) according to any one of claims 1 to 5, 8, 9 and 15, the semi-finished tape material (60) according to any one of claims 6 to 12 and 15 or hygiene item (70) according to claim 13 or 15, **characterised in that** the first adhesive functional region (31) lies between 2.5 and 10 mm wide and/or the second adhesive functional region (32) lies between 10 mm and 30 mm and/or that a width ratio between the first and the second adhesive functional region (31, 32) lies between 1:2 and 1:5.

17. The hygiene item closure tape (10) according to any one of claims 1 to 5, 8, 9, 15 and 16, the semi-finished tape material (60) according to any one of claims 6 to 12, 15 and 16 or hygiene item (70) according to claim 13 to 16, **characterised in that** at least the rear side (14), preferably the entire carrier tape (11), comprises an imprint.

18. The hygiene item closure tape (10) according to any one of claims 1 to 5, 8, 9, 15 to 17, the semi-finished tape material (60) according to any one of claims 6 to 12 and 15 to 17 or hygiene item (70) according to claims 13 to 17, **characterised in that** the adhesive (12) of at least one of the two adhesive functional regions (31, 32) is profiled, wherein the profiling preferably has a profile depth, which amounts to at least half the thickness of the adhesive (12) and/or wherein the profiling has at least one profile valley with a minimum valley diameter of at least the thickness of the adhesive (12) or of at least 0.5 mm.

19. The hygiene item closure tape (10) according to any one of claims 1 to 5, 8, 9, 15 to 18, the semi-finished tape material (60) according to any one of claims 6 to 12 and 15 to 18 or hygiene item (70) according to claim 13 or 18, **characterised in that** the rear side (14) carries means for reducing and/or increasing the static friction and in particular is siliconised.

## Revendications

1. Bande de fermeture (10) adhésive d'un article d'hygiène, pourvue d'une bande de support (11), laquelle comporte une face avant (13) portant un agent adhésif (12) et une face arrière (14), sur la face avant (13) étant prévue au moins une couche d'agent adhésif (41, 42), qui constitue une première zone fonctionnelle adhésive (31) et une deuxième zone fonctionnelle adhésive (32), **caractérisée en ce que** des forces de décollage de la première zone fonctionnelle adhésive (31) des deux zones fonctionnelles adhésives (31, 32) sont supérieures à des forces de décollage de la deuxième zone fonctionnelle adhésive (32) des deux zones fonctionnelles adhésives (31, 32), la première zone fonctionnelle adhésive (31) des deux zones fonctionnelles adhésives (31, 32) constituant une zone permanente, la zone permanente (29) servant à fixer de manière permanente la bande de fermeture (10) d'un article d'hygiène sur un article d'hygiène (70), et la deuxième zone fonctionnelle adhésive (32) des deux zones fonctionnelles adhésives (31, 32) constituant une zone dédiée à l'utilisateur, la zone dédiée à l'utilisateur (28) servant à offrir à un utilisateur la possibilité d'ouvrir et de fermer la fermeture constituée par la bande de fermeture (10) d'un article d'hygiène et à ce que la première zone fonctionnelle adhésive (31) soit constituée en étant plus petite que la deuxième zone fonctionnelle adhésive (32).

2. Bande de fermeture (10) d'un article d'hygiène selon la revendication 1, **caractérisée en ce que** sur la face avant (13) sont prévues deux couches (41, 42) d'agent adhésif, une première couche d'agent adhésif (41) des deux couches (41, 42) d'agent adhésif constituant la première zone fonctionnelle adhésive (31) et une deuxième couche d'agent adhésif (42) des deux couches (41, 42) d'agent adhésif constituant la deuxième zone fonctionnelle adhésive (32), les deux couches (41, 42) d'agent adhésif étant placées de préférence en étant directement voisines ou même en se chevauchant.

3. Bande de fermeture (10) d'un article d'hygiène selon la revendication 1 ou 2, **caractérisée en ce que** la bande de fermeture (10) d'un article d'hygiène s'étend d'un pied (15) jusqu'à une tête (16), le long d'une direction (51) d'extension longitudinale.

4. Bande de fermeture (10) d'un article d'hygiène selon la revendication 3, **caractérisée en ce qu'**au moins l'une des deux zones fonctionnelles adhésives (31, 32) arrive à la perpendiculaire de la direction (51) d'extension longitudinale, d'une arête (17) de la bande de fermeture (10) d'un article d'hygiène vers une autre arête (17) de la bande de fermeture (10) d'un article d'hygiène.

5. Bande de fermeture (10) d'un article d'hygiène selon la revendication 3 ou 4, **caractérisée en ce que** dans la direction (51) d'extension longitudinale, la première zone fonctionnelle adhésive (31) est conçue en étant plus étroite que la deuxième zone fonctionnelle adhésive (32).

6. Produit semi-fini (60) en bande, destiné à fabriquer une bande de fermeture (10) d'un article d'hygiène s'étendant le long d'une direction (51) d'extension longitudinale orientée à la perpendiculaire d'une direction (52) d'extension de la bande du produit semi-fini (60) en bande, notamment selon l'une quelconque des revendications 1 à 5, pourvu d'une bande de support (11), laquelle comporte une face avant (13) portant un agent adhésif (12) et une face arrière (14), sur la face avant (13) étant prévu au moins un ruban (61, 62) d'agent adhésif, qui constitue une première zone fonctionnelle adhésive (31) et une deuxième zone fonctionnelle adhésive (32), **caractérisé en ce que** des forces de décollage de la première zone fonctionnelle adhésive (31) des deux zones fonctionnelles adhésives (31, 32) sont supérieures aux forces de décollage de la deuxième zone fonctionnelle adhésive (32) des deux zones fonctionnelles adhésives (31, 32), la première zone fonctionnelle adhésive (31) des deux zones fonctionnelles adhésives (31, 32) constituant une zone permanente, la zone permanente (29) servant à fixer de manière permanente la bande de fermeture (10) d'un article d'hygiène sur un article d'hygiène (70) et la deuxième zone fonctionnelle adhésive (32) des deux zones fonctionnelles adhésives (31, 32) constituant une zone dédiée à l'utilisateur, la zone dédiée à l'utilisateur (28) servant à offrir à un utilisateur la possibilité d'ouvrir et de fermer la fermeture constituée par la bande de fermeture (10) d'un article d'hygiène et à ce que la première zone fonctionnelle adhésive (31) soit constituée en étant plus étroite que la deuxième zone fonctionnelle adhésive (32) dans la direction (51) d'extension longitudinale.

7. Produit semi-fini (60) en bande selon la revendication 6, **caractérisé en ce que** sur la face avant (13) sont prévus deux rubans (61, 62) d'agent adhésif, un premier ruban (61) d'agent adhésif des deux rubans (61, 62) d'agent adhésif constituant la première zone fonctionnelle adhésive (31) et un deuxième ruban (62) d'agent adhésif des deux rubans (61, 62) d'agent adhésif constituant la deuxième zone fonctionnelle adhésive (32), les deux couches (41, 42) d'agent adhésif étant placées de préférence en étant directement voisines ou même en se chevauchant.

8. Bande de fermeture (10) d'un article d'hygiène selon l'une quelconque des revendications 1 à 5 ou produit semi-fini (60) en bande selon la revendication 6 ou 7, caractérisé(e) en ce que les forces de décollage de la première zone fonctionnelle adhésive (31) sont supérieures du double, de préférence de deux fois et demies aux forces de décollage de la deuxième zone fonctionnelle adhésive (32).

9. Bande de fermeture (10) d'un article d'hygiène selon l'une quelconque des revendications 1 à 5 et 8 ou produit semi-fini (60) en bande selon l'une quelconque des revendications 6 à 8, caractérisé(e) par exactement une seule première zone fonctionnelle adhésive (31) et exactement une seule deuxième zone fonctionnelle adhésive (32).

10. Produit semi-fini (60) en bande selon l'une quelconque des revendications 6 à 8, **caractérisé en ce qu'**il est constitué en étant à usages multiples et **en ce qu'**un ruban (61) d'agent adhésif constituant une première zone fonctionnelle adhésive (31) est placé entre deux rubans (62) d'agent adhésif constituant des deuxièmes zones fonctionnelles adhésives (32).

11. Produit semi-fini (60) en bande selon la revendication 10, **caractérisé en ce que** de part et d'autre des deux rubans (62) d'agent adhésif constituant les deuxièmes zones fonctionnelles adhésives (32) est placé chaque fois un ruban (65), destiné à constituer une zone fonctionnelle (25) supplémentaire de la bande de fermeture (10) d'un article d'hygiène.

12. Produit semi-fini (60) en bande selon la revendication 10 ou 11, **caractérisé en ce que** le ruban (61) d'agent adhésif constituant la première zone fonctionnelle adhésive (31) ne s'étend pas plus de 20 mm dans la direction d'extension longitudinale.

13. Article d'hygiène (70), fermé ou susceptible d'être fermé à l'aide d'une bande de fermeture (10) d'un article d'hygiène selon l'une quelconque des revendications 1 à 5, 8 et 9, comprenant une première zone de fermeture (71) et une deuxième zone de fermeture (72), qui doivent être fermées l'une avec l'autre, et la bande de fermeture (10) d'un article d'hygiène, laquelle par sa première zone fonctionnelle adhésive (31) constituant une zone permanente (29) est assemblée, susceptible d'être assemblée ou assemblée de manière amovible avec la première zone de fermeture (71) et par sa deuxième zone fonctionnelle adhésive (32) constituant une zone dédiée à l'utilisateur (28) est assemblée, susceptible d'être assemblée ou assemblée de manière amovible avec la deuxième zone de fermeture (72), **caractérisé en ce que** les deux zones fonctionnelles adhésives (31, 32) de la bande de fermeture (10) d'un article d'hygiène sont susceptibles d'être assemblées ou sont assemblées conjointement avec la première zone de fermeture (71).

14. Article d'hygiène (70) selon la revendication 13, **caractérisé en ce que** la première zone de fermeture (71) et / ou la deuxième zone de fermeture (72) sont prévues sur un film d'une épaisseur de 25 µm ou inférieure ou d'une épaisseur moindre à celle de la bande de support (11).

15. Bande de fermeture (10) d'un article d'hygiène selon l'une quelconque des revendications 1 à 5, 8 et 9, produit semi-fini (60) en bande selon l'une quelconque des revendications 6 à 12 ou article d'hygiène (70) selon la revendication 13 ou 14, caractérisé(e) en ce que la première zone fonctionnelle adhésive (31) et la deuxième zone fonctionnelle adhésive (32) ne sont pas écartées l'une de l'autre de plus de 1 mm.

16. Bande de fermeture (10) d'un article d'hygiène selon l'une quelconque des revendications 1 à 5, 8, 9 et 15, produit semi-fini (60) en bande selon l'une quelconque des revendications 6 à 12 et 15 ou article d'hygiène (70) selon la revendication 13 ou 15, caractérisé(e) en ce que la première zone fonctionnelle adhésive (31) présente une largeur comprise entre 2,5 et 10 mm et / ou en ce que la deuxième zone fonctionnelle adhésive (32) présente une largeur comprise entre 10 mm et 30 mm et /ou en ce qu'un rapport de largeur de la première à la deuxième zones fonctionnelles adhésives (31, 32) se situe entre 1 : 2 et 1 : 5.

17. Bande de fermeture (10) d'un article d'hygiène selon l'une quelconque des revendications 1 à 5, 8, 9, 15 et 16, produit semi-fini (60) en bande selon l'une quelconque des revendications 6 à 12, 15 et 16 ou article d'hygiène (70) selon la revendication 13 à 16, caractérisé(e) en ce qu'au moins la face arrière (14), de préférence l'ensemble de la bande de support (11) comporte un gaufrage.

18. Bande de fermeture (10) d'un article d'hygiène selon l'une quelconque des revendications 1 à 5, 8, 9 et 15 à 17, produit semi-fini (60) en bande selon l'une quelconque des revendications 6 à 12 et 15 à 17 ou article d'hygiène (70) selon la revendication 13 à 17, caractérisé(e) en ce que l'agent adhésif (12) d'au moins l'une des deux zones fonctionnelles adhésives (31, 32) est profilé, le profilage présentant de préférence une profondeur de profil qui s'élève à au moins la moitié de l'épaisseur de l'agent adhésif (12) et / ou le profilage comportant de préférence un creux de profil d'un diamètre minimum de creux correspondant au moins à l'épaisseur de l'agent adhésif (12) ou d'au moins 0,5 mm.

19. Bande de fermeture (10) d'un article d'hygiène selon l'une quelconque des revendications 1 à 5, 8, 9 et 15 à 18, produit semi-fini (60) en bande selon l'une quelconque des revendications 6 à 12 et 15 à 18 ou article d'hygiène (70) selon la revendication 13 à 18, caractérisé (e) en ce que la face arrière (14) porte des moyens destinés à réduire et / ou à augmenter le frottement statique et est notamment siliconée.
